# EUROPEAN PATENT APPLICATION

(11) **EP 3 594 955 A1**
(43) Date of publication of application: **15.01.2020**
(21) Application number: 18183365.8
(22) Date of filing: 13.07.2018
(51) Int. Cl.: G16H 10/60, G16H 40/67, G06Q 50/00

(54) **PATIENT INFORMATION SYSTEM**

(71) Applicant: Dalin Tzu Chi Hospital, Buddhist Tzu Chi Medical Foundation, 62247 Chiayi County (TW)
(72) Inventor: CHENG, Po-Liang, 62247 Dalin Township, Chiayi County (TW)
(74) Representative: Viering, Jentschura & Partner mbB Patent- und Rechtsanwälte

(57) **Abstract**

A patient information system includes a patient information sharing platform (10), a terminal device (20), and an identification label (30). The patient information sharing platform (10) is configured for storing a patient data (11) and a patient status data (12). When a communication sensing module (21) of the terminal device (20) is connected with the identification label (30) through a sensing connection, the terminal device (20) is configured for acquiring or editing the patient data (11) and the patient status data (12). Therefore, the patient related information is efficiently acquired by the user (3).

## Description

### BACKGROUND OF THE INVENTION

### 1. Field of the Invention:

The present invention relates to information systems, and more particularly, to a patient information system.

### 2. Description of the Related Art:

In a large scale accident, medical staff from various hospitals arrive the scene of the accident for rescuing a large amount of patients. A medical personnel has to efficiently identify the identity of each patient, so as to provide different treatment procedures according to the different injury levels of the patients.

Regarding a conventional procedure for handling the large amount of patients, the medical staff usually identify the patient identities and carry out the medical tag registration manually, and provide treatments according to different medical examination levels. However, manually identifying the patient identities not only prolongs the rescue duration, but also easily cause mistaken identification and registrations. As a result, medical malpractice might occur due to the misidentification of patients.

Also, patient information acquired by manual medical examination and medical tag registration are unable to be shared for all medical staff. Therefore, a single patient might undergo the same identification process by different medical personnel, delaying the rescuing procedure and causing a waste of resource.

Further, before a large amount of patients are delivered to the hospital, the medical staff in the hospital have to be generally informed about the patient amount and the scale of the accident according to the information from the medical personnel at the scene of the accident. However, the medical staff in the hospital are unable to know the injury detail of each patient. Thus, when a large amount of patients, the medical staff in the hospital have to confirm the patient identity and carry out the medical examination again. Also, the medical staff in the hospital are unable to know if the patient has received an initial treatment at the scene of the accident. As a result, the medical staff in the hospital face a chaotic situation, and the medical treatment efficiency is lowered.

### SUMMARY OF THE INVENTION

For improving the issues above, a patient information system is disclosed by the present invention. By use of the sensing connection between the terminal device and the identification label, the patient information is timely updated, facilitating a highly efficient treatment process.

A patient information system in accordance with an embodiment of the present invention comprises:
a patient information sharing platform set up on a server, the patient data sharing platform storing a patient data and a patient status data;
a terminal device connected with the patient information sharing platform through the internet, the terminal device comprising a communication sensing module; and
an identification label providing a sensing identification function and an data transaction function, wherein when the identification label is sensed to be connected with the communication sensing module, the terminal device is able to acquire and edit the patient data and the patient status data.

With such configuration, when an accident occurs, the medical staff are allowed to identify the patient identities at the scene of the accident, and then establish the patient data and patient status data in the patient information sharing platform. Afterward, the medical staff in the subsequent state are able to efficiently identify and confirm the treatment status of the patients through the terminal device, achieving a highly efficient treatment process.

In an embodiment of the present invention, the patient information sharing platform includes a location module, and the terminal device includes a positioning module. The positioning module acquires a current location signal of the terminal device, and the terminal device transmits the current location signal which represents the current location of the patient to the location module, wherein the location module transmits the current location module to the patient stats information. Therefore, the commander at the scene of the accident is able to known the locations of each medical personnel and each patient according to the current location signal displayed on the location module, thereby determining a support needed location, facilitating an efficient rescue.

In an embodiment of the present invention, the patient information sharing platform comprises a hospital data module, and the hospital data module has a plurality of treatment end information. The patient status data has one of the corresponding treatment end information. Therefore, the commander at the scene of the accident is allowed to arrange each patient to receive treatment at a suitable hospital based the treatment end information, the amount of patients, and the respective injury levels. Further, family members of the patients are able to know the hospital accepting the target patient according to the patient status data.

### BRIEF DESCRIPTION OF THE DRAWINGS

**Fig. 1** is a block diagram illustrating the connection of the patient information system in accordance with an embodiment of the present invention.
**Fig. 2** is a schematic view illustrating the system structure of the patient information system in accordance with an embodiment of the present invention.
**Fig. 3** is a schematic view illustrating the user applying the terminal device to sense the identification label.

### DETAILED DESCRIPTION OF THE INVENTION

The aforementioned and further advantages and features of the present invention will be understood by reference to the description of the preferred embodiment in conjunction with the accompanying drawings where the components are illustrated based on a proportion for explanation but not subject to the actual component proportion.

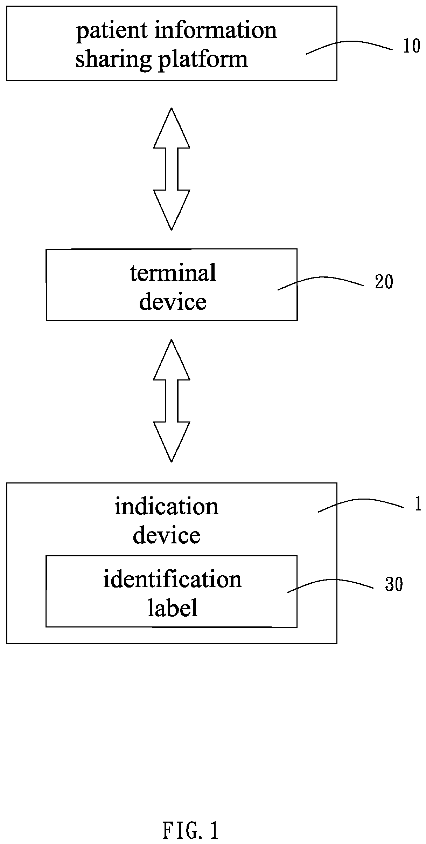

Referring to **Fig. 1 to Fig. 3**, a patient information system in accordance with an embodiment of the present invention comprises a patient information sharing platform **10**, a terminal device **20**, and an identification label **30.** The patient information sharing platform **10** is set up on a server, and providing access to at least one user **3** through the internet, such that the user **3** is able to access the patient information sharing platform **10** by use of the terminal device **20.** The terminal device **20** is a portable device, such as a smart phone or a table computer.

Also, the patient information system further includes an identification label **30**, which provides a sensing identification function and a data transaction function. The identification label **30** is disposed on an indication device **1**, which is worn on the patient **2.** In an embodiment of the present invention, the indication device **1** is a wristband, and the identification label **30** is an NFC (near field communication) label.

In addition, the terminal device **20** includes a communication sensing module **21.** When the communication sensing module **21** is connected with the identification label **30** on the indication device **1** by use of the near field communication. The terminal device **20** is allowed to acquire or edit the patient data **11** and the patient status data **12** stored on the patient information sharing platform **10.** In the embodiment of the present invention, the user **3** wears the indication device **1** on the patient **2**, wherein the user **3** is allowed to be a medical personnel, the rescue commander, one of a medical staff in the hospital, a medical personnel on the ambulance, or a non-medical personnel (such as a family member of the patient **2**).

The patient information sharing platform **10** comprises a patient data module **13**, a location module **14**, a hospital data module **15**, an account module **16**, and an authorization module **17.**

The patient data module **13** stores the patient data **11** and the patient status data **12.** The patient data **11** includes an identification data, a treatment record data, and a pattern corresponding to the identification label **30.** In an embodiment of the present invention, when the user **3** who is a medical personnel wears the indication device **1** on the patient **2**, the user **3** senses the identification label **30** through the terminal device **20** with a sensing connection, such that the user **3** is allowed to establish the patient data **11** and patient status data **12** in the patient data module **13** through the terminal device **20.** Also, the user **3** saves the identification label **30** corresponding to the indication device **1** in the patient data **11.** The patient data **11** is allowed to be chosen from a text, image, video, or combination thereof. Also, the patient data **11** is allowed to include identification related data, such as the name, gender, and age of the patient **2.** The patient status data **12** is allowed to be chosen from a text, image, video, or combination thereof.

The location module **14** is configured to display the location related information. In an embodiment of the present invention, the terminal device **20** includes a positioning module **22** for acquiring a current location signal of the terminal device **20.** The positioning module **22** refers to a GPS module. The terminal device **20** transmits the current location signal to the location module **14**, such that the location module **14** displays the current location signal of the terminal device **20.** Also, the location module **14** is able to transmit the current location signal to the patient status data **12.**

The hospital data module **15** includes a plurality of treatment end information **151**, wherein the treatment end information **151** includes hospital related information, such as the name and classification of the hospital. In an embodiment of the present invention, the user **3** is able to arrange the patient **2** to be delivered to a suitable hospital according to the injury status of the patient **2**, and the user **3** is also able to use the terminal device **20** to establish the treatment end information **151** arranged for the patient **2** in the patient status data **12.**

The account module **16** allows the user to register a user information **161** with the account module **16** through the terminal device **20.**

The authorization module **17**, according to the user information **161** in the account module **16**, limits the authority of the user **3** viewing the data on the patient information sharing platform **10.** More specifically, when the user **3** is a non-medical personnel, the authorization module **17** imposes the limitation upon the viewable data content for the user **3.** For example, the user **3** is thus only allowed to view the patient status data **12** or the identification data in the patient data **11.** Further, when the user **3** is a medical personnel, the user **3** is allowed to view all related data of the patient **2**, including the patient status data **12**, patient data **11**, and the location displayed by the location module **14.**

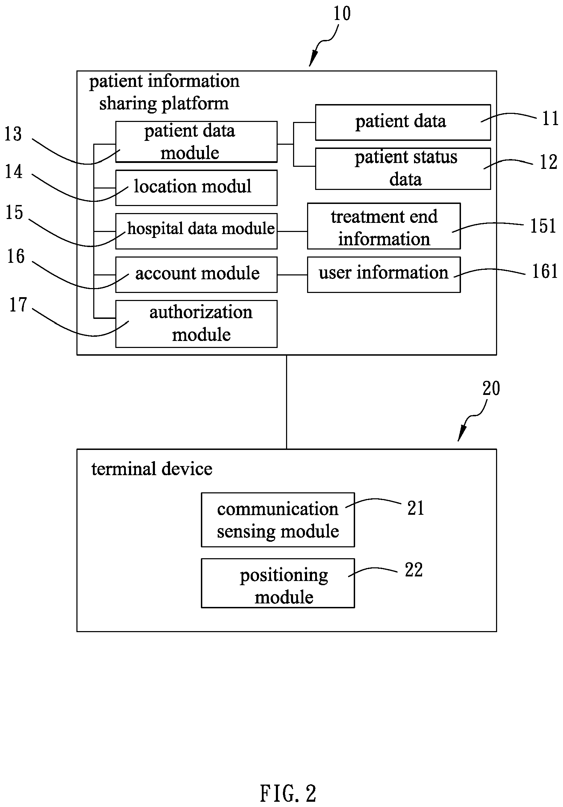

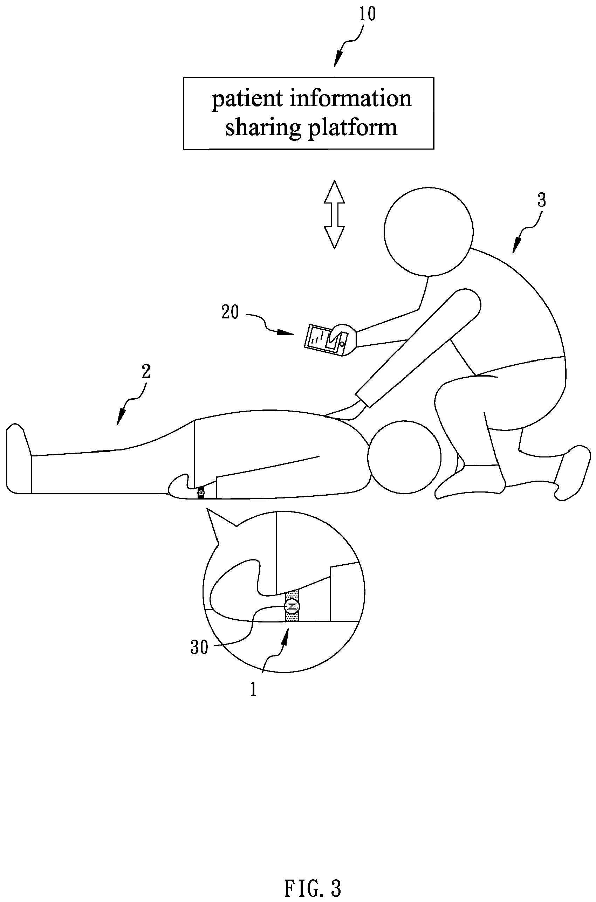

Referring to **Fig. 2** and **Fig. 3**, when an accident occurs, the user **3** may be a medical personnel or a rescue commander. The medical personnel wears the indication device **1** on the patient **2**, confirms the identity of the patient **2**, and carries out a medical examination upon the patient **2.** Next, the medical personnel establishes the identity and injury status of the patient **2** in the patient data **11** and patient status data **12** through the terminal device **20.**

The rescue commander is then allowed to use the terminal device **20** to acquire the identity and the injury status of each patient **2** at the scene of accident. Also, based on the current location signal transmitted from the positioning module **22** of the terminal device **20** to the location module **14**, the rescue commander is able to know the location of each medical personnel and patient **2**, so as to determine the rescue needed locations.

Further, according to each treatment end information **151** and the amount and injury levels of the patients **2**, the rescue commander is able to arrange each patient **2** to be delivered to the suitable hospital for receiving treatment, and also establish the treatment end information **151** arranged for the patient **2** in the patient status data **12.** The medical staff in the hospital are allowed to use the terminal device **20** for acquiring the patient data **11** and patient status data **12** of the patients **2** from the patient status data **12** in the patient information sharing platform **10** in advance. Also, the family member of a respective patient **2** is able to acquire the treatment end information **151** arranged for the patient **2** from the patient status data **12** in the patient information sharing platform **10** through the terminal device **20.**

When the patient **2** is delivered to the hospital, the medical staff in the hospital are able to efficiently identify and confirm the treatment status of the patient **2** through the terminal device **20**, thus providing a suitable treatment process, facilitating a highly efficient treatment.

Although particular embodiments of the invention have been described in detail for purposes of illustration, various modifications and enhancements may be made without departing from the spirit and scope of the invention. Accordingly, the invention is not to be limited except as by the appended claims.

## Claims

1. A patient information system comprising:
a patient information sharing platform (10) set up on a server for storing a patient data (11) and a patient status data (12);
a terminal device (20) connected with the patient information sharing platform (10) through the internet, the terminal device (20) comprising a communication sensing module (21); and
an identification label (30) providing a sensing identification function and an data transaction function, wherein when the identification label (30) is sensed to be connected with the communication sensing module (21), the terminal device (20) is able to acquire and edit the patient data (11) and the patient status data (12).

2. The patient information system of claim 1, wherein the identification label (30) is disposed on an indication device (1) worn on a patient (2), the communication sensing module (21) connected with the identification label (30) through a near field communication connection.

3. The patient information system of claim 2, wherein the patient data (11) includes the identification label (30) assigned to the patient (2).

4. The patient information system of claim 1, wherein the patient information sharing platform (10) comprises a patient data module (13), the patient data module (13) stores the patient data (11) and the patient status data (12).

5. The patient information system of claim 1, wherein the patient data (11) comprises an identification data and a treatment record data.

6. The patient information system of claim 1, wherein the patient information sharing platform (10) comprises a location module (14), and the terminal device (20) comprises a positioning module (22), such that the positioning module (22) acquires a current location signal of the terminal device (20), and the terminal device (20) transmits the current location signal to the location module (14).

7. The patient information system of claim 6, wherein the location module (14) transmits the current location signal to the patient status data (12).

8. The patient information system of claim 1, wherein the patient information sharing platform (10) comprises a hospital data module (15), the hospital data module (15) comprises a plurality of treatment end information (151), the patient status data (12) has one of the corresponding treatment end information (151).

9. The patient information system of claim 1, wherein the patient information sharing platform (10) comprises an account module (16), the terminal device (20) provides access for an user (3) to operate the patient information sharing platform (10) through the internet, and the user (3) registers a user information (161) with the account module (16).

10. The patient information system of claim 9, wherein the patient information sharing platform (10) comprises an authorization module (17), the authorization module (17) limits an authority of the user (3) for viewing data on the patient information sharing platform (10) according to the user information (161) in the account module (16).
